# EUROPEAN PATENT APPLICATION

(11) **EP 2 634 576 A1**
(43) Date of publication of application: **04.09.2013**
(21) Application number: 12157763.9
(22) Date of filing: 01.03.2012
(51) Int. Cl.: G01N 33/52

(54) **Method for the preparation of a dry composition comprising a stable ABTS radical**

(71) Applicant: Universiteit Maastricht, 6211 LK Maastricht (NL); Academisch Ziekenhuis Maastricht, 6229 HX Maastricht (NL)
(72) Inventor: Balk, Jiska Mirjam, 6229 ER Maastricht (NL); Bast, Aalt, 3862 XG Nijkerk (NL); Haenen, Guido Rembertus Michiel Marie, 6211 GJ Maastricht (NL)
(74) Representative: Habets, Winand

(57) **Abstract**

The invention is in the field of medical and nutritional diagnostics. It provides means and methods for determining the antioxidant status of a sample. The invention provides a method for obtaining a stable radical ABTS in solid form wherein ABTS is dissolved in a buffered aqueous solution at a pH between 7 and 8, converted into radical ABTS, frozen and freeze dried.

## Description

### Field of the invention

The invention is in the field of diagnostics in particular medical or nutritional diagnostics. It provides means and methods for determining the antioxidant status of a sample.

### Background of the invention

Free radicals and reactive oxygen species (ROS) are highly reactive molecules that are generated by normal cellular processes, environmental stresses, and UV irradiation. ROS react with cellular components, damaging DNA, carbohydrates, proteins, and lipids causing cellular and tissue injury. Excess production of reactive oxygen species can also lead to inflammation, premature aging disorders, and several disease states, including cancer, diabetes, and atherosclerosis. Organisms have developed complex antioxidant systems to protect themselves from oxidative stress, however, excess ROS can overwhelm the systems and cause severe damage.

Oxidative stress is widely recognized as a factor in many degenerative diseases, as either a cause or effect. The atherosclerotic plaques that obstruct arterial flow and cause cardiovascular disease are laid down by macrophages engorged with oxidized LDL (foam cells). Oxidized bases in DNA are potentially mutagenic and are therefore implicated in the process of carcinogenesis. Diabetes mellitus is associated with oxidative damage to biomolecules, and any inflammatory condition inevitably leads to an increased oxidative burden, because the release of reactive oxygen species by macrophages is part of the bodys defense mechanism (8).

An antioxidant is a molecule capable of inhibiting the oxidation of other molecules. Oxidation is a chemical reaction that transfers electrons or hydrogen from a substance to an oxidizing agent. Oxidation reactions can produce free radicals. In turn, these radicals can start chain reactions. When the chain reaction occurs in a cell, it can cause damage or death to the cell. Antioxidants terminate these chain reactions by removing free radical intermediates, and inhibit other oxidation reactions. They do this by being oxidized themselves, so antioxidants are often reducing agents such as thiols, ascorbic acid, or polyphenols.

Although oxidation reactions are crucial for life, they can also be damaging; plants and animals maintain complex systems of multiple types of antioxidants, such as glutathione, vitamin C, and vitamin E as well as enzymes such as catalase, superoxide dismutase and various peroxidases. Low levels of antioxidants, or inhibition of the antioxidant enzymes, cause oxidative stress and may damage or kill cells.

As oxidative stress appears to be an important part of many human diseases, the use of antioxidants in pharmacology is intensively studied, particularly as treatments for stroke and neurodegenerative diseases. However, it is unknown whether oxidative stress is the cause or the consequence of disease.

Antioxidants are widely used as ingredients in dietary supplements and have been investigated for the prevention of diseases such as cancer and coronary heart disease. When antioxidant supplementation is tested for their effectiveness in patients, the quality of life benefits. In addition to these uses of antioxidants in medicine, these compounds have many industrial uses, such as preservatives in food and cosmetics and preventing the degradation of rubber and gasoline.

Antioxidants are found in varying amounts in foods such as vegetables, fruits, grain cereals, eggs, meat, legumes and nuts. Some antioxidants such as lycopene and ascorbic acid can be destroyed by long-term storage or prolonged cooking. Other antioxidant compounds are more stable, such as the polyphenolic antioxidants in foods such as whole-wheat cereals and tea. The effects of cooking and food processing are complex, as these processes can also affect the bioavailability of antioxidants, such as some carotenoids in vegetables. In general, processed foods contain less antioxidants than fresh and uncooked foods.

Besides antioxidants that are derived from food sources, like vitamins, the human body can also synthesize antioxidants. For example, ubiquinol (coenzyme Q) is poorly absorbed from the gut and is made in humans. Another example is glutathione, which is made from amino acids. Any glutathione is broken down in the gut to free cysteine, glycine and glutamic acid before being absorbed. Large amounts of sulfur-containing amino acids such as N-acetylcysteine can increase glutathione. Supplying more of these precursors may be useful as part of the treatment of some diseases, such as acute respiratory distress syndrome, protein-energy malnutrition, or preventing the liver damage produced by paracetamol overdose.

Other compounds in the diet can alter the levels of antioxidants indirectly, by enhancing the endogenous production of antioxidants. Some of these compounds, such as isothiocyanates and curcumin, may be chemopreventive agents that either block the transformation of abnormal cells into cancerous cells, or even kill existing cancer cells.

The role of antioxidants obtained from the diet in protection against disease is a topic of continuing interest. Several studies revealed beneficial effects of antioxidants from the diet, for example the effect of n-acetylcysteine on COPD patients (van Herwaarden, Netherlands journal of medicine, 1995).

An alternative, the molecular epidemiologic approach, has several advantages, notably the small number of subjects needed and the short time scale. Its success depends, however, on the use of reliable validated biomarkers, which are rare. A notable example involves chromosomal aberrations, which were shown in prospective studies to be good indicators of future risk of cancer (5, 6). DNA damage is clearly the ultimate cause of cancer, because DNA base changes can be mutagenic, but, except for some special cases (7), DNA damage is probably better regarded as a marker of exposure to genotoxic agents than as an indicator of the likelihood that cancer will occur in an individual.

The sum of endogenous and food-derived antioxidants represents the total antioxidant activity of the extracellular fluid.

Cooperation of different antioxidants provides greater protection against oxidative stress. Thus, the overall antioxidant capacity gives more relevant biological information compared to that obtained by the measurement of individual components, as it considers the cumulative effect of all antioxidants present in plasma and body fluids.

Measurement of antioxidants is not a straightforward process, as this is a diverse group of compounds with different reactivities to different reactive oxygen species. Several antioxidant capacity assays have been developed and are used for testing. Each test has specific characteristics and limitations. The debate about a standard measurement is still ongoing. Previous research revealed that, from the specific oxidative stress biomarkers, the TEAC is the most sensitive biomarker (9).

There are several other assays described and on the market that measure anti-oxidant status. The Trolox Equivalent Antioxidant Assay measures the total antioxidant capacity of plasma, serum, urine, saliva or other solutions/fluids. The assay relies on the ability of antioxidants to reduce the ABTS radical (2,2'-azino-di- [3-ethylbenzothiazoline-sulphonate] radical).The capacity of the antioxidants in the sample to reduce the ABTS radical is compared with that of Trolox, a water soluble tocopherol analogue, and is quantified as Trolox equivalents (10). This assay however can only be performed in the laboratory, using specialized equipment.

There is still a need in the art for reliable easy-to-use assays that measure the antioxidant status of a sample. The present invention addresses that need.

### Summary of the invention

The invention provides a method for obtaining a stable radical ABTS in solid form wherein ABTS is dissolved in a buffered aqueous solution at a pH between 7 and 8, converted into radical ABTS, frozen and freeze dried.

The invention also relates to a stable radical ABTS in solid form obtainable by that method.

The invention also relates to a solid support comprising an immobilized radical ABTS.

The invention also relates to the use of such a solid support for determining the antioxidant status of a sample.

### Detailed description of the invention

We found that ABTS (2,2-azino-bis-3 ethylbenzothiazoline-6-sulfonic acid) may be used in a simple and reliable ready-for-use test in order to determine the antioxidant status of a sample. For that purpose, ABTS is dissolved in a buffered aqueous solution at a pH between 7 and 8. ABTS is then converted into radical ABTS, frozen and freeze dried.

Contrary to the prejudice in the art that radical ABTS is not stable, we were able to obtain a stable ABTS radical powder upon freeze drying of a radical ABTS solution.

The thus obtained radical ABTS powder may be used to determine the antioxidant status of a sample. For instance by immobilizing the ABTS radical on a solid support. This may be accomplished by contacting the ABTS radical solution with a solid support such as a boron silica strip. The solid support comprising the radical ABTS solution is then frozen and freeze dried in order to obtain a test strip.

When the radical ABTS radical powder is contacted with a sample containing antioxidants, the color of the powder changes from green to colourless.

The assay was found to be sensitive in the micromolar range, i.e. it provided reliable results between 1 micromolar and 1 millimolar of ascorbic acid in solution.

The test strip can be used in nutritional and clinical research for quick testing of the antioxidant capacity of many samples. In a representative test, the sample is contacted with the test strip and the degree of decolorization is quantified. In a preferred embodiment, the test strip is dipped into the sample fluid, and the degree of decolorization is quantified.

General practitioners and analysts in clinical chemistry departments can use this test strip for a quick determination of the antioxidant status of patients. The sample is therefore placed on the test strip and the degree of decolorization is then quantified. Preferred samples for measuring the antioxidant status are blood, saliva and urine.

In food production, the test strip can be used to test the quality of ingredients and products. It can also be used to test the shelf life of the products. The sample is placed on the test strip and the degree of decolorization is quantified. Otherwise, the test strip is dipped into the sample fluid, and the degree of decolorization is quantified.

Consumers can use the test strip at home to measure the quality of their antioxidant containing products. Potentially, it can also be used to assess their own health status. The sample is placed on the test strip and the degree of decolorization is quantified. Otherwise, the test strip is dipped into the sample fluid, and the degree of decolorization is quantified.

### Examples

### Example 1: preparation of ABTS radical powder

ABTS (2,2-azino-bis-3 ethylbenzothiazoline-6-sulfonic acid) was dissolved in a 145 mM sodiumphosphate buffer, pH 7.4 at room temperature at a concentration of 10 mM. Four units of horseradish peroxidase were added to the solution and hydrogen peroxide was subsequently added to reach a concentration of 70 nM (ABTS radical solution). It was observed that the solution obtained a dark green color within 10 minutes.

The solution was then kept at -80 degrees Celsius overnight and then freeze dried to obtain ABTS radical powder.

### Example 2: Preparation of ABTS antioxidant strips.

Boron silica strips were rinsed with the dark green ABTS radical solution. The strips were then placed in sealed tubes and placed at -80 degrees Celsius overnight. The strips were freeze dried while in the tubes for several days. The strips were then stored in a water free environment.

### Example 3. Use of ABTS antioxidant strips to determine the antioxidant status of a sample.

The ABTS powder and ABTS strips were tested for their reactivity with several concentrations of ascorbic acid in a buffer, ranging from 1 microMolar to 1 milliMolar. Therefore, the test strips were contacted with the different concentrations of ascorbic acid for 1 minute. Decolorization was observed after 10 minutes. The results are displayed in the table below.

It was found that the decolorization of the ABTS radical powder on the test strip was proportional to the concentration of ascorbic acid.

| Concentration ascorbic acid [micro Molar] | Color of ABTS test strip |
|---|---|
| 0 | Bright green |
| 1 | Bright green |
| 10 | Bright green |
| 50 | Light green |
| 100 | very faint green |
| 1000 | Colorless |

### References

1. Alpha-Tocopherol, Beta-Carotene Cancer Prevention Study Group. N Engl J Med 1994;330:1029-35.
2. Omenn GS, Goodman GE, Thornquist MD, et al. N Engl J Med 1996;334:1150-5.
3. Hennekens CH, Buring JE, Manson J, et al. N Engl J Med 1996;334:1145-9.
4. Heart Protection Study Collaborative Group. Lancet 2002;360:23-33.
5. Hagmar L, Brøgger A, Hansteen I-L, et al. Cancer Res 1994;54:2919-22.
6. Bonassi S, Abbondandolo A, Camurri L, et al. Cancer Genet Cytogenet 1998;79:133-5.
7. Poirier MC. Environ Health Perspect 1997;105(suppl 4):907-12.
8. Collins, A. Am. J. Clin. Nutr. 2005; 81: 2615-2675.
9. Berg van den, R et al., J. Nutr. 2001; 131: 1714-1722.
10. Fischer, M et al., Clin. Chem. Lab. Med. 2005; 43: 735-740.

## Claims

1. Method for obtaining a stable radical ABTS in solid form comprising the steps of dissolving ABTS in a buffered aqueous solution at a pH between 7 and 8, converting the dissolved ABTS into radical ABTS, freezing the thus obtained radical ABTS solution and freeze drying the frozen solution to obtain radical ABTS in solid form.

2. A stable radical ABTS in solid form obtainable by the method according to claim 1.

3. A solid support comprising an immobilized radical ABTS according to claim 2.

4. Use of a solid support according to claim 3 for determining the antioxidant status of a sample.

5. Use according to claim 4 wherein the sample is a sample derived from a human individual.

6. Use according to claim 5 wherein the sample is a blood, plasma or serum sample.
